# EUROPEAN PATENT SPECIFICATION

(11) **EP 2 214 642 B1**
(45) Date of publication and mention of the grant of the patent: **03.05.2017**
(21) Application number: 08844931.9
(22) Date of filing: 27.10.2008
(51) Int. Cl.: A61K 9/00, A61K 31/196, A61K 9/06, A61K 9/107

(54) **TOPICAL COMPOSITION**
TOPISCHE ZUSAMMENSETZUNG
COMPOSITION TOPIQUE

(30) Priority: 30.10.2007 EP 07119599
(43) Date of publication of application: 11.08.2010
(73) Proprietor: Novartis Consumer Health S.A., 1197 Prangins (CH)
(72) Inventor: CAILLET-BOIS, Fabienne, CH-1869 Massongex (CH); RAULT, Isabelle, F-01170 Segny (FR); STEIGER, Michel, CH-1814 La Tour-de-Peilz (CH)
(74) Representative: Reeves, Julie Frances
(86) International application number: PCT/EP2008/064533
(87) International publication number: WO 2009/056522

(56) References cited:
- EP-A- 0 879 597
- EP-A- 1 457 202
- WO-A-00/51575
- WO-A-2004/030665
- US-A- 4 917 886

## Description

The invention concerns topical formulations comprising the well-known and widely used non-steroidal anti-inflammatory drug (NSAID) diclofenac in emulsion-gel form. The currently commercially most successful product of this kind is Voltaren® Emulgel® comprising 1.16% diclofenac diethylamine salt (corresponding to 1% diclofenac sodium).

It was an object of the present invention to provide an emulsion-gel with similarly advantageous properties as Voltaren® Emulgel® but with even improved efficacy in conditions like e.g. back pain, osteoarthritis, or muscle orjoint injuries, and allowing less frequent applications of the product per day.

EP1 457 202 (Yung Shin Pharm Ind Co Ltd) discloses a topical formulation containing diclofenac for treating the skin of patients with pain associated with lesions/blisters around the lip area caused by the herpes virus. The "emulgel" of Example 6 does not contain either a lipid or a surfactant.

WO2004/030665 (Thalas Group Inc) is limited to the sodium salt of diclofenac and teaches how to obtain a transparent gel by combining it with oleyl and isopropyl alcohol, providing a topical, local or systemic effect.

US 4,917,886 (Ashe and Affolter) - see also below- discloses emulsion gels comprising NSAIDs. Only the sodium salt of diclofenac is disclosed in Examples 1-4.

WO00/51575 proposes transdermal devices comprising NSAIDs in which the NSAID is dissolved in an autoadhesive matrix. It is not the object of this matrix to yield therapeutic efficacy.

It turned out quickly that said object could not be reached by simply increasing the concentration of diclofenac diethylammonium salt. When doing so, the two main issues observed, among others, were an incomplete dissolution of the higher amounts of active substance used and an insufficient penetration through the skin of the higher amounts of active substance used. It was found that it is crucial for diclofenac to be fully dissolved (with no identifiable crystals of diclofenac being present in the formulation) rather than partly suspended. Only then a high, constant and reproducible permeation of diclofenac through the skin is guaranteed. A further issue, as it turned out, was that said improved topical formulation, as any commercial pharmaceutical product, had to be stable over time, that is to say have a sufficiently long shelf life.

The inventors of the present invention ran a lot of experiments to test various diclofenac concentrations, various diclofenac salts and various additional excipients to resolve these and other issues. As an interim result, the following formulations were obtained (see Table 1):

**Table 1: Cumulative permeation in vitro of 20 mg/cm² of products on human skin (in 1µg/cm²)**

| Time [h] | Voltaren® Emulgel® | Emulsion-gel 2.32% DEA¹ 2% glycerin monolaurate | | Emulsion-gel 2.32% DEA¹ 2% oleic acid | | Emulsion-gel 2.32% DEA¹ 2% oleyl alcohol | |
|---|---|---|---|---|---|---|---|
| | mean | mean | n-ford increase | mean | n-fold increase | mean | n-fold increase |
| 0 | 0.00 | 0.00 | 0.00 | 0.00 | 0.00 | 0.00 | 0.00 |
| 2 | 0.30 | 0.53 | 1.77 | 0.35 | 1.16 | 0.36 | 1.22 |
| 4 | 0.83 | 1.26 | 1.51 | 0.75 | 0.90 | 1.18 | 1.42 |
| 8 | 1.73 | 3.80 | 2.19 | 3.49 | 2.02 | 3.82 | 2.21 |
| 24 | 6.65 | 13.51 | 2.03 | 20.47 | 3.08 | 18.33 | 2.76 |
| 32 | 8.81 | 18.40 | 2.09 | 28.19 | 3.20 | 24.11 | 2.74 |

| | | | | | | | |
|---|---|---|---|---|---|---|---|
| ¹ DEA = diclofenac diethylammonium salt | | | | | | | |

Three emulsion-gels similar to Voltaren® Emulgel® but each including 2.32% of diclofenac diethylammonium salt and further 2% of either glycerin monolaurate, oleic acid or oleyl alcohol, turned out to include the active diclofenac in fully dissolved state and, moreover, provided a dramatically increased permeation of diclofenac through the skin, both as desired.

However, when examining said three formulations further, it turned out that the formulation containing 2% of glycerin monolaurate was not suitable, as it did not show sufficient stability of the emulsion over time. What the formulation containing 2% of oleic acid was concerned, it did not have a sufficiently high viscosity, even when maximum possible amounts of gelling agents (carbomers) were applied. Moreover, when its microscopic aspect was evaluated, it turned out that the droplets forming the emulsion were too big, which bears the risk of an undesired separation of the two phases of the emulsion (unmixing). What is desired and needed is to have fine droplets upon microscopic inspection.

A further hurdle to overcome before completing the invention was local skin tolerance: The local skin tolerance of Voltaren® Emulgel® is known to be very good, i.e. the appearance of skin irritations after application is very rare, and also the systemic toxicity of said product is very low. A goal for the emulsion-gel of the present invention was to come as close as possible to the safety profile set by Voltaren® Emulgel®.

After a lot of experimentation including many failures the inventors succeeded in obtaining emulsion-gels fulfilling all of the mentioned requirements. Therefore the invention relates to a topical pharmaceutical composition, which is in the form of an opaque emulsion-gel and comprises 1.2-4% (w/w) of diclofenac diethylammonium salt;
(A) said composition having a high skin permeation,
(B) said composition showing a very low systemic absorption only,
(C) said composition showing essentially no irritation on human skin after administration, and
(D) said composition being chemically and physically stable when stored at 25°C and a relative humidity of 60% for 12 months.

Thus, the topical pharmaceutical preparations of the present invention exhibit various beneficial properties, as further outlined below.
(A) Having high skin permeation means e.g. an in vitro cumulative permeation on human skin that is at 24h at least twice that of "reference product A" defined as follows: "Reference product A" is the pharmaceutical formulation disclosed in Example 1 of US patent 4,917,886 (Asche and Affolter).
Moreover, the high skin permeation can be seen from the fact that the topical preparations of the invention show an in vitro cumulative permeation on human skin at 24h which is at least twice that of commercial product Voltaren® Emulgel®, see Table 2.

**Table 2: Cumulative permeation in vitro of 20 mg/cm² of products on human skin (in 1µg/cm²)**

| Time [h] | Voltaren® Emulgel® | Emulsion-gel with 2.32% DEA¹ and 0.5% oleyl alcohol (= Example 1a) | | Emulsion-gel with 2.32% DEA¹ and 0.75% oleyl alcohol (= Example 1) | | Emulsion-gelwith 2.32% DEA¹ and 1.0% oleyl alcohol (= Example 1b) | |
|---|---|---|---|---|---|---|---|
| | mean | mean | n-fold increase | mean | n-fold increase | mean | n-fold increase |
| 0 | 0.00 | 0.00 | 0.00 | 0.00 | 0.00 | 0.00 | 0.00 |
| 2 | 0.04 | 0.04 | 0.9 | 0.08 | 1.7 | 0.04 | 0.8 |
| 4 | 0.20 | 0.35 | 1.8 | 0.26 | 1.3 | 0.23 | 1.2 |
| 8 | 0.43 | 0.56 | 1.3 | 0.78 | 1.8 | 0.74 | 1.7 |
| 24 | 2.07 | 4.92 | 2.4 | 6.11 | 3.0 | 5.95 | 2.9 |

| | | | | | | | |
|---|---|---|---|---|---|---|---|
| ¹ DEA = diclofenac diethylammonium salt | | | | | | | |

This in vitro test is able to provide suitable relative values (in comparison to a reference formulation). But that it does not provide reproducible absolute values, can be seen e.g. from the following partial repetition of the above experiments (see Table 3), where the absolute values for both Voltaren® Emulgel® and the formulation of Example 1 turned out to be higher than in Table 2.

**Table 3: Cumulative permeation in vitro of 20 mg/cm² of products on human skin (in 1µg/cm²)**

| Time [h] | Voltaren® Emulgel® | Emulsion-gel with 2.32% DEA¹ and 0.75% oleyl alcohol (= Example 1) | |
|---|---|---|---|
| | mean | mean | n-fold increase |
| 0 | 0.00 | 0.00 | 0.0 |
| 2 | 0.06 | 0.27 | 4.5 |
| 4 | 0.37 | 1.20 | 3.2 |
| 8 | 1.72 | 5.63 | 3.3 |
| 24 | 9.27 | 23.70 | 2.6 |

In an analogous test series, high skin penetration, similar to oleyl alcohol, was also obtained with 1-dodecanol, 1-tetradecanol and 1-octadecanol (see Table 4).

**Table 4: Cumulative permeation in vitro of 20 mg/cm² of products on human skin (in 1µg/cm²)**

| Time [h] | Emulsion-gel with 2.32% DEA¹ and 0.75% oleyl alcohol (=Example 1) | Emulsion-gelwith 2.32% DEA¹ and 0.75% 1-octadecanol (= Example 4) | | Emulsion-gel with 2.32% DEA¹ and 0.75% 1-tetradecanol (= Example 5) | | Emulsion-gel with 2.32% DEA¹ and 0.75% 1-dodecanol (= Example 3) | |
|---|---|---|---|---|---|---|---|
| | mean | mean | ratio v. Example 1 | mean | ratio v. Example 1 | mean | ratio v. Example 1 |
| 0 | 0.00 | 0.00 | 0.0 | 0.00 | 0.0 | 0.00 | 0.0 |
| 2 | 2.18 | 1.56 | 0.7 | 1.98 | 0.9 | 3.59 | 1.6 |
| 4 | 6.09 | 5.62 | 0.9 | 6.23 | 1.0 | 9.58 | 1.6 |
| 8 | 16.38 | 13.98 | 0.9 | 17.59 | 1.1 | 26.43 | 1.6 |
| 24 | 53.02 | 43.44 | 0.8 | 57.60 | 1.1 | 72.93 | 1.4 |

(B) Very low systemic absorption absorption: Although having high skin permeation, the topical preparations of the invention show only a very low systemic absorption, which means an AUC value (area under curve from t = 0 toT= 12h) of less than 30 ng x hour/ml- preferably less than 25, more preferably less than 20, and especially less than 15 ng x hour/ml - derived from a pharmacokinetic graph "diclofenac concentration (in the blood) versus time" after one application of 2g thereof to a body area of 400 cm². "Body area", preferably means the knee but numeric values indicated are likewise valid for other body areas, e.g. ankle or elbow.
(C) Essentially no irritation on human skin after administration: Moreover, their cutaneous tolerance upon intended use in humans is very good. This was demonstrated e.g. in a challenging in vivo test on rabbits which received a daily 4h administration of the product over 28 days, only very slight irritation was observed (see also Test example 3). The results of said tests can be summarized such that the compositions of the invention (C) essentially show no irritation on human skin after administration.
(D) Chemical and physical stability: They are chemically and physically stable, which means having a shelf life of at least 12 months, preferably at least 24 months, when stored at 25°C and at a relative humidity of 60%. Likewise, they proved to be stable for at least 6 months when stored at 40°C and at a relative humidity of 75%.
Chemical and physical stability further means that (i) that the emulsion-gel structure of the composition was maintained without breaking of the emulsion, and (ii) that the original color of the composition did not visibly change, e.g. via yellowing, over a period of at least 12 months when stored at 25°C and at a relative humidity of 60%.
(E) Full dissolution of diclofenac diethylammonium salt: Moreover, the active substance, diclofenac diethylammonium salt, is kept fully dissolved (in the emulsion-gel structure of the composition), which means that even upon microscopic examination no crystals of diclofenac diethylammonium salt could be observed therein.
(F) Higher pain relief: Moreover, the topical pharmaceutical preparations of the present invention provide higher pain relief than other commercially available topical diclofenac preparations comprising 1.16% of diclofenac diethylammonium salt or 1.0% of diclofenac sodium, respectively, such as Voltaren® Emulgel®, or than reference product A as defined hereinbefore. This can e.g. be demonstrated in comparative tests on patients suffering e.g. from osteoarthritis. Whereas said known commercially available topical diclofenac preparations require at least four applications per day to control pain, the present invention provides a method of controlling pain in a human being suffering from osteoarthritis, which method is characterized in that a topical pharmaceutical composition of claim 1 is administered to said human being only once or twice daily, preferably twice daily.

Preferably, the amount of diclofenac diethylammonium salt in the compositions of the invention is 1.7-4%, in particular 2-4%.

A saturated or unsaturated C1O-C18 fatty alcohol is e.g. oleyl alcohol, stearyl alcohol, myristyl alcohol or lauryl alcohol. Typically, it is present in an amount of from 0.5 up to 1.5%

preferably of from 0.5 up to 1% and in particular of from 0.7 up to 1% of the total composition. Preferred is oleyl alcohol.

The topical pharmaceutical preparations of the present invention comprise the components (c), (d), (e), (f), (g), (h)
and (i) as specified for the various embodiments below.

An embodiment of the invention is characterized by a topical pharmaceutical composition, which is in the form of an opaque emulsion-gel, and which comprises
(a) 1.2-4% (w/w) of diclofenac diethylammonium salt,
(b) 0.5-2% (w/w) of a saturated or unsaturated C10-C18 fatty alcohol selected from the group consisting of stearyl alcohol, myristyl alcohol, lauryl alcohol and oleyl alcohol,
(c) at least 40% (w/w) of water,
(d) 10-30% (w/w) of at least one C2-C4-alkanol,
(e) 3-15% (w/w) of at least one glycol solvent selected from the group consisting of 1,2-propanediol and polyethylene glycol (200-20000),
(f) 0.5-5% (w/w) of at least one gelling agent selected from the group consisting of carbomers,
(g) 2-8% (w/w) of at least one liquid lipid forming the oily phase of the emulsion-gel,
(h) 1-3% (w/w) of at least one non-ionic surfactant, and
(i) a basic agent to adjust the pH of the total composition to 6-9.

Preferably, diclofenac diethylammonium salt (a) is the only pharmaceutically active substance being present in the composition.

All percentages given are percentages by weight (w/w), if not indicated otherwise.

Preferred are those compositions that comprise
(a) 1.5-3.5% of diclofenac diethylammonium salt,
(b) 0.5-2%, preferably 0.5-1.5%, of oleyl alcohol,
(c) 45-75% of water,
(d) 10-30% of ethanol, isopropanol, or mixtures thereof,
(e) 3-12% of 1,2-propanediol,
(f) 0.7-3% of at least one gelling agent selected from the group consisting of carbomers,
(g) 3-7% of at least one liquid lipid forming the oily phase of the emulsion-gel,
(h) 1-3% of at least one non-ionic surfactant, and
(i) 0.5-2% of diethylamine to adjust the pH of the total composition to 6.5-8.5.

Preferably, diclofenac diethylammonium salt (a) is present in an amount of from 1.3 up to 3.5% more preferably of from 1.5 up to 3.5%, especially of from 1.7 up to 3% and in particular of from 2 up to 2.5% of the total composition, or in an amount of from 1.7 up to 4%, in particular of from 2 up to 4%, of the total composition.

Preferably, the saturated or unsaturated C1O-C18 fatty alcohol selected from the group consisting of stearyl alcohol, myristyl alcohol, lauryl alcohol and oleyl alcohol, (b), is present in an amount of from 0.5 up to 1.5% preferably of from 0.5 up to 1% and in particular of from 0.7 up to 1% of the total composition. Preferred is oleyl alcohol.

Water (c) is preferably present in an amount of from 45 up to 75%, preferably of from 50 up to 75%, especially of from 55 up to 75% and in particular of from 60 up to 70%, of the total composition.

Preferred as C2-C4-alkanols (d) are ethanol, isopropanol, or mixtures thereof; and in particular isopropanol. Typically, (d) is present in an amount of from 10 up to 30%, preferably of from 10 up to 25%, especially of from 12 up to 23% and in particular of from 15 up to 20%, of the total composition.

Preferably, the glycol solvent (e) is 1,2-propanediol. Alternatively, polyethylene glycol (200-20000), e.g. polyethylene glycol (200-1000), may also be used as (e). The glycol solvent (e) is preferably present in an amount of from 3 up to 12%, more preferably of from 3 up to 10%, especially of from 3 up to 8%, more especially of from 3 up to 7%, and in particular of from 3.5 up to 6%, of the total composition.

Carbomers (f), in the context of the present invention, are defined as homo- or copolymers of acrylic acid that are cross-linked, e.g. with an allyl ether of pentaerythritol (allyl pentaerythritol) or an allyl ether of sucrose (allyl sucrose). Copolymers are formed e.g. with minor levels of long chain alkyl acrylate co-monomers. Homopolymers are preferred.

Especially preferred are carbomers 980, 940, 981, 941, 974, 934 and 910.1n particular preferred are the following products provided by Noveon, Inc, Cleveland, Ohio, USA (formerly B F Goodrich): Carbopol® 980 and Carbopol® 974- especially Carbopol® 980, and analogous carbomer products from other suppliers. Preferably, (f) is present in an amount of from 0.7 up to 3%, more preferably of from 0.8 up to 2%, especially of from 0.9 up to 1.8% and in particular of from 0.9 up to 1.6%, of the total composition.

The liquid lipid (g) forms the oily phase of the emulsion-gels of the invention. It can be of a vegetable or animal nature, or partly or completely synthetic. There come into consideration lipids without ester linkages, e.g. hydrocarbons, and lipids having ester linkages, e.g. glycerides - i.e. fatty acid esters of glycerol -, especially triglycerides, or esters of fatty acids, e.g. with C1-C36-alkanols, especially C8-C36-alkanols. Hydrocarbons are e.g. paraffin or petroleum jelly. Glycerides are e.g. olive oil, castor oil, sesame oil, it being possible for all said oils also to be hydrogenated; caprylic/capric acid triglyceride or glycerol mono-, di- and tri-esters with palmitic and/or stearic acid. Esters of fatty acids with C1-C36-alkanols are e.g. beeswax, carnauba wax, cetyl palmitate, lanolin, isopropyl myristate, isopropyl stearate, oleic acid decyl ester, ethyl oleate, or C6-C12-alkanoic acid esters - especially caprylic/capric acid esters-with saturated fatty alcohols, especially C12-C18 saturated fatty alcohols.

Preferably, the liquid lipid (g) comprises C6-C12-alkanoic acid C12-C18-alkyl esters. In particular preferred is a mixture of liquid paraffin and C6-C12-alkanoic acid C12-C18-alkyl esters- especially caprylic/capric acid esters with C12-C18 saturated fatty alcohols (coco-caprylate/caprate, e.g. Cetioi®LC). The liquid lipid(s) (g) are preferably present in a total amount of from 3 up to 7%, especially of from 4 up to 6%, of the total composition. If a mixture of paraffin and coco-caprylate/caprate is used, the amount of paraffin is preferably 1.5-3%, especially 2-2.8%, and the amount of coco-caprylate/caprate is preferably 1.5-3%, especially 2-2.8%, of the total composition.

(h) A non-ionic surfactant is e.g. an ester of a fatty acid, especially a C8-C18 fatty acid, with monohydroxy or, preferably, polyhydroxy compounds, e.g. ethylene glycol, glycerol, anhydrosorbitol or pentaerythritol. Another important group of non-ionic surfactants is represented by the poly(oxyethylated) surfactants, which mean compounds that have at least one active hydrogen, e.g. fatty alcohols- especially a C8-C18 fatty alcohol, fatty acids especially a C8-C18 fatty acid, sorbitan fatty acid esters, C1-C18-alkylphenols or C8-C18-alkylamines, and that all are poly(oxyethylated), preferably with from 2 up to 40 ethylene glycol or ethylene oxide units.

Examples for the above mentioned non-ionic surfactants are partial glycerin fatty acid esters, such as glycerin monostearate; partial fatty acid esters of sorbitan or polyoxyethylene sorbitan, such as sorbitan monolaurate or polyethylene glycol (5 to 20) sorbitan monostearate or monooleate; polyoxyethylene (3 to 40) fatty alcohol ethers, such as polyoxyethylene (3 to 12) lauryl ethers or polyoxyethylene (5 to 40) cetostearyl ethers; polyoxyethylene fatty acid esters, such as polyoxyethylene (8 to 100) stearate; polyoxyethylene C4-C12-alkylphenyl ethers, e.g. polyoxyethylene (nonyl or octyl)phenyl ethers; or polyoxyethylene C8-C18-alkylamines, e.g. polyoxyethylene oleylamine. Preferred are polyoxyethylene (10 to 30) fatty alcohol ethers, in particular polyoxyethylene (20) cetostearyl ether (e.g. Cetomacrogol 1000). Typically, the non-ionic surfactant (g) is present in an amount of from 1 up to 3%, preferably of from 1.5 up to 2.5%, of the total composition.

The basic agent (i) to adjust the pH of the total composition to 6-9 especially 6.5-8 is preferably diethylamine. In case that diethylamine is used, it is e.g. present in an amount of 0.5-2%, especially 0.7-1.6%, of the total composition. In general, (i) can be present e.g. in amount of from 0.1 up to 10% of the total composition.

The compositions of the inventions may optionally include further routine excipients known in the art, for example fragrances/perfumes, antioxidants, e.g. butylhydroxytoluene, antimicrobial preservatives, e.g. benzyl alcohol, benzalkonium chloride or parabens (= C1-C7-alkyl esters of 4-hydroxybenzoic acid, e.g. methyl 4-hydroxybenzoate), and coloring agents.

The topical pharmaceutical compositions according to the invention are administered in a manner known per se. For example, they are applied e.g. once or twice daily to the affected portions of the skin.

The invention further relates to a method of treating inflammatory diseases including pain which comprises topically administering to a mammal in need of such treatment a therapeutically effective amount of one of the topical pharmaceutical compositions as specified herein above and below.

The manufacture of the topical pharmaceutical preparations is effected in a manner known per se, for example as described in the examples below.

The following examples are intended to illustrate the invention.
Example 1: An emulsion gel comprising 2.32% of diclofenac diethylammonium salt

| Ingredients | Amount (kg/10Qkg) |
|---|---|
| (a) Diclofenac diethylammonium salt | 2.32 |
| (b) Oleyl alcohol | 0.75 |
| (c) Purified water | 64.26 |
| (d) Isopropanol | 17.50 |
| (e) 1,2-Proponediol (= Propylene glycol) | 5.00 |
| (f) Carbomer 980 | 1.70 |
| (g') Paraffin, liquid | 2.50 |
| (g") Coco-caprylate/caprate | 2.50 |
| (h) Polyoxyethylene-20-cetostearyl ether | 2.00 |
| (i) Diethylamine | 1.35 |
| Buthylhydroxytoluene (BHT) | 0.02 |
| Perfumes | 0.10 |
| | 100.0 |

Manufacture: (a) is dissolved in (d), (e) and part of (c). Said solution is added to a mixture of the remainder of (c) and (f) which is neutralized by adding (i). All remaining components-(g'), (g") and (h), (b), BHT and perfumes- are heated and slowly added to the former mixture. Upon mixing a homogeneous emulsion-gel is obtained.
Example 1a: The ingredients of Example 1 are slightly modified by using 0.5% oleyl alcohol and 64.51% of water (instead of 0.75% oleyl alcohol and 64.26% of water). Thereby, an emulsion gel comprising 2.32% of diclofenac diethylammonium salt and 0.5% oleyl alcohol is obtained.
Example 1b: The ingredients of Example 1 are slightly modified by using 1.0% oleyl alcohol and 64.01% of water (instead of 0.75% oleyl alcohol and 64.26% of water). Thereby, an emulsion gel comprising 2.32% of diclofenac diethylammonium salt and 0.5% oleyl alcohol is obtained.

Example 2: An emulsion gel comprising 3.48% of diclofenac diethylammonium salt

| Ingredients | Amount (kg/100kg) |
|---|---|
| (a) Diclofenac diethylammonium salt | 3.48 |
| (b) Oleyl alcohol | 1.00 |
| (c) Purified water | 49.35 |
| (d) Isopropanol | 25.00 |
| (e) 1,2-Propanediol | 10.00 |
| (f) Carbomer 980 (g') Paraffin, liquid | 2.00 |
| (g") Coca-caprylate/caprate | 2.50 |
| (h) Polyoxyethylene-20-cetostearyl ether | 2.50 |
| (i) Diethylamine | 1.55 |
| Butylhydroxytoluene | 0.02 |
| Perfumes | 0.10 |
| | 100.0 |

Manufacture: As described in Example 1.
Example 3: An emulsion gel comprising 2.32% of diclofenac diethylammonium salt: The composition and manufacture is the same as in Example 1, with the exception that 0.75 kg/100kg of lauryl alcohol (1-dodecanol) are used (b) instead of 0.75 kg/100kg oleyl alcohol.
Example 4: An emulsion gel comprising 2.32% of diclofenac diethylammonium salt: The composition and manufacture is the same as in Example 1, with the exception that 0.75 kg/100kg of stearyl alcohol (1-octadecanol) are used (b) instead of 0.75 kg/100kg oleyl alcohol.
Example 5: An emulsion gel comprising 2.32% of diclofenac diethylammonium salt: The composition and manufacture is the same as in Example 1, with the exception that 0.75 kg/100kg of myristyl alcohol (1-tetradecanol) are used (b) instead of 0.75 kg/100kg oleyl alcohol.

Test examples 1: The stability of the emulsion gel of Example 1 was tested via an assay of diclofenac diethylammonium salt. In doing so, the formulation was stored under various conditions (temperature/relative humidity) and for various storage times, and at the end the amount of diclofenac diethylammonium salt still being present was determined. The results were as follows:

| Storage time / Condition | 25°C/60% r.h. | 30°C/75% r.h. | 40°C/75% r.h. |
|---|---|---|---|
| 20g tubes: start | 100.1% | n.a. | n.a. |
| 20g tubes: 3 months | 100.2% | 100.4% | 100.6% |
| 20g tubes: 6 months | 99.7% | 99.4% | 100.6% |
| 20g tubes: 12 months | 99.5% | 99.8% | not tested |
| 100g tubes: start | 100.6% | n.a. | n.a. |
| 1OOg tubes: 3 months | 100.8% | 100.5% | 100.9% |
| 1OOg tubes: 6 months | 100.6% | 100.4% | 100.4% |
| 100g tubes: 12 months | 99.7% | 99.8% | not tested |

| | | | |
|---|---|---|---|
| (n.a. = not applicable) | | | |

It was demonstrated that the active substance is completely stable even under demanding storage conditions for long periods of time.

Test example 2 (microscopic examination): The emulsion gel of Example 1 was examined under 100x magnification and scrutinized for the presence of any crystals of diclofenac diethylammonium salt. Absolutely no, not even tiny, crystals of diclofenac diethylammonium salt are observed. One only sees the very fine droplets of the emulsion.

Test example 3 [local skin tolerance test in vivo (in rabbits, n = 6)]: After daily application for 4 hours during 28 consecutive days, the cutaneous tolerance of the emulsion gel of Example 1 proved to be very good.

## Claims

1. A topical pharmaceutical composition, which is in the form of an opaque emulsion-gel, and which comprises
(a) 1.2-4% (w/w) of diclofenac diethylammonium salt,
(b) 0.5-2% (w/w) of a saturated or unsaturated C10-C18 fatty alcohol selected from the group consisting of stearyl alcohol, myristyl alcohol, lauryl alcohol and oleyl alcohol;
(c) at least 40% (w/w) of water,
(d) 10-30% (w/w) of at least one C2-C4-alkanol,
(e) 3-15% (w/w) of at least one glycol solvent selected from the group consisting of 1,2-propanediol and polyethylene glycol (200-20000),
(f) 0.5-5% (w/w) of at least one gelling agent selected from the group consisting of carbomers,
(g) 2-8% (w/w) of at least one liquid lipid forming the oily phase of the emulsion-gel,
(h) 1-3% (w/w) of at least one non-ionic surfactant, and
(i) a basic agent to adjust the pH of the total composition to 6-9.

2. A composition according to claim 1, wherein the saturated or unsaturated C10-C18 fatty alcohol (b) is oleyl alcohol.

3. A composition according to claim 1 or claim 2, which comprises
(a) 1.5-3.5% of diclofenac diethylammonium salt,
(b) 0.5-2% oleyl alcohol,
(c) 45-75% of water,
(d) 10-30% of ethanol, isopropanol, or mixtures thereof.
(e) 3-12% of 1,2-propanediol,
(f) 0.7-3% of at least one gelling agent selected from the group consisting of carbomers,
(g) 3-7% of at least one liquid lipid forming the oily phase of the emulsion-gel,
(h) 1-3% of at least one non-ionic surfactant, and
(i) 0.5-2% of diethylamine to adjust the pH of the total composition to 6.5-8.5.

4. A composition according to claim 3, wherein the oleyl alcohol (b) is present in an amount of from 0.6 up to 1.2% of the total composition.

5. A composition according to any one of claims 1-4, wherein the component (d) is isopropanol.

6. A composition according to any one of claims 1-5, wherein the liquid lipid (g) is selected from the group consisting of hydrocarbons, glycerides, esters of fatty acids and any mixtures thereof.

7. A composition according to any one of claims 1-6, wherein the non-ionic surfactant (h) is selected from the group consisting of esters of fatty acids with monohydroxy or polyhydroxy compounds and poly(oxyethylated) surfactants and any mixtures thereof.

8. A composition according to any one of claims 1-7 for use in controlling pain in a human being suffering from osteoarthritis, wherein said composition is administered to said human being only once or twice daily.

## Patentansprüche

1. Topische pharmazeutische Zusammensetzung, die in Form eines opaken Emulsionsgels vorliegt und die umfasst:
(a) 1,2 bis 4 % (G/G) eines Diclofenac-Diethylammoniumsalzes,
(b) 0,5 bis 2 % (G/G) eines gesättigten oder ungesättigten C₁₀-₁₈-Fettalkohols, ausgewählt aus der Gruppe bestehend aus Stearylalkohol, Myristylalkohol, Laurylalkohol und Oleylalkohol,
(c) zumindest 40 % (G/G) Wasser,
(d) 10 bis 30 % (G/G) zumindest eines C₂₋₄-Alkanols,
(e) 3 bis 15 % (G/G) zumindest eines Glykol-Lösungsmittels, ausgewählt aus der Gruppe bestehend aus 1,2-Propandiol und Polyethylenglykol (200-20.000),
(f) 0,5 bis 5 % (G/G) zumindest eines Geliermittels, ausgewählt aus der Gruppe bestehend aus Carbomeren,
(g) 2 bis 8 % (G/G) zumindest eines flüssigen Lipids, das die ölige Phase des Emulsionsgels bildet,
(h) 1 bis 3 % (G/G) zumindest eines nicht-ionischen Tensids und
(i) ein basisches Mittel zur Einstellung des pH-Wertes der gesamten Zusammensetzung auf 6 bis 9.

2. Zusammensetzung gemäss Anspruch 1, wobei der gesättigte oder ungesättigte C₁₀₋₁₈-Fettalkohol (b) Oleylalkohol ist.

3. Zusammensetzung gemäss Anspruch 1 oder Anspruch 2, umfassend:
(a) 1,5 bis 3,5 % Diclofenac-Diethylammoniumsalz,
(b) 0,5 bis 2 % Oleylalkohol,
(c) 45 bis 75 % Wasser,
(d) 10 bis 30 % Ethanol, Isopropanol oder Mischungen davon,
(e) 3 bis 12 % 1,2-Propandiol,
(f) 0,7 bis 3 % zumindest eines Gelierungsmittels, ausgewählt aus der Gruppe bestehend aus Carbomeren,
(g) 3 bis 7 % zumindest eines flüssigen Lipids, das die ölige Base des Emulsionsgels bildet,
(h) 1 bis 3 % zumindest eines nicht-ionischen Tensids und
(i) 0,5 bis 2 % Diethylamin zur Einstellung des pH-Wertes der gesamten Zusammensetzung auf 6,5 bis 8,5.

4. Zusammensetzung gemäss Anspruch 3, wobei der Oleylalkohol (b) in einer Menge von 0,6 bis zu 1,2 % der gesamten Zusammensetzung vorhanden ist.

5. Zusammensetzung gemäss irgendeinem der Ansprüche 1 bis 4, wobei die Komponente (d) Isopropanol ist.

6. Zusammensetzung gemäss irgendeinem der Ansprüche 1 bis 5, wobei das flüssige Lipid (g) aus der Gruppe bestehend aus Kohlenwasserstoffen, Glyceriden, Fettsäureestern und irgendeiner Mischung davon ausgewählt ist.

7. Zusammensetzung gemäss irgendeinem der Ansprüche 1 bis 6, wobei das nicht-ionische Tensid (h) aus der Gruppe bestehend aus Estern von Fettsäuren mit Monohydroxy- oder Polyhydroxyverbindungen und poly(oxyethylierten) Tensiden und irgendeiner Mischung davon ausgewählt ist.

8. Zusammensetzung gemäss irgendeinem der Ansprüche 1 bis 7 zur Verwendung bei der Bekämpfung von Schmerzen bei einem Menschen, der an Osteoarthritis leidet, wobei die Zusammensetzung nur ein- oder zweimal täglich an den Menschen verabreicht wird.

## Revendications

1. Composition pharmaceutique topique, qui est sous la forme d'une émulsion-gel opaque, et qui comprend
(a) 1,2 à 4 % (p/p) de sel de diéthylammonium de diclofénac,
(b) 0,5 à 2 % (p/p) d'un alcool gras en C10 à C18 saturé ou insaturé choisi dans le groupe constitué de l'alcool stéarylique, l'alcool myristylique, l'alcool laurylique et l'alcool oléylique,
(c) au moins 40 % (p/p) d'eau,
(d) 10 à 30 % (p/p) d'au moins un alcanol en C2 à C4,
(e) 3 à 15 % (p/p) d'au moins un solvant de glycol choisi dans le groupe constitué du 1,2-propanediol et de polyéthylène glycol (200 à 20 000),
(f) 0,5 à 5 % (p/p) d'au moins un agent gélifiant choisi dans le groupe constitué de carbomères,
(g) 2 à 8 % (p/p) d'au moins un lipide liquide formant la phase huileuse de l'émulsion-gel,
(h) 1 à 3 % (p/p) d'au moins un tensioactif non ionique, et
(i) un agent basique pour ajuster le pH de la composition totale à 6 à 9.

2. Composition selon la revendication 1, dans laquelle l'alcool gras en C10 à C18 saturé ou insaturé (b) est l'alcool oléylique.

3. Composition selon la revendication 1 ou la revendication 2, qui comprend
(a) 1,5 à 3,5 % de sel de diéthylammonium de diclofénac,
(b) 0,5 à 2 % d'alcool oléylique,
(c) 45 à 75 % d'eau,
(d) 10 à 30 % d'éthanol, d'isopropanol, ou des mélanges de ceux-ci,
(e) 3 à 12 % de 1,2-propanediol,
(f) 0,7 à 3 % d'au moins un agent gélifiant choisi dans le groupe constitué de carbomères,
(g) 3 à 7 % d'au moins un lipide liquide formant la phase huileuse de l'émulsion-gel,
(h) 1 à 3 % d'au moins un tensioactif non ionique, et
(i) 0,5 à 2 % de diéthylamine pour ajuster le pH de la composition totale à 6,5 à 8,5.

4. Composition selon la revendication 3, dans laquelle l'alcool oléylique (b) est présent dans une quantité de 0,6 jusqu'à 1,2 % de la composition totale.

5. Composition selon l'une quelconque des revendications 1 à 4, dans laquelle le composant (d) est l'isopropanol.

6. Composition selon l'une quelconque des revendications 1 à 5, dans laquelle le lipide liquide (g) est choisi dans le groupe constitué d'hydrocarbures, de glycérides, d'esters d'acide gras et de l'un quelconque de leurs mélanges.

7. Composition selon l'une quelconque des revendications 1 à 6, dans laquelle le tensioactif non ionique (h) est choisi dans le groupe constitué d'esters d'acides gras avec des composés monohydroxylés ou polyhydroxylés et des tensioactifs poly(oxyéthylés) et de l'un quelconque de leurs mélanges.

8. Composition selon l'une quelconque des revendications 1 à 7 pour une utilisation dans le contrôle de la douleur chez un être humain souffrant d'arthrose, ladite composition étant administrée au dit être humain seulement une fois ou deux fois par jour.
